# EUROPÄISCHE PATENTSCHRIFT

(11) **EP 3 573 787 B1**
(45) Veröffentlichungstag und Bekanntmachung des Hinweises auf die Patenterteilung: **29.03.2023**
(21) Anmeldenummer: 18702210.8
(22) Anmeldetag: 25.01.2018
(51) Int. Cl.: B25B 15/00, A61B 17/88

(54) **ACHSGENAUER SCHRAUBENDREHER**
AXIALLY PRECISE SCREWDRIVER
TOURNEVIS À PRÉCISION AXIALE

(30) Priorität: 25.01.2017 DE 102017101348
(43) Veröffentlichungstag der Anmeldung: 04.12.2019
(73) Patentinhaber: Aesculap AG, 78532 Tuttlingen (DE)
(72) Erfinder: FISCHER, Kay, 78532 Tuttlingen (DE); LINDNER, Stephan, 78573 Wurmlingen (DE)
(74) Vertreter: Winter, Brandl - Partnerschaft mbB
(86) Internationale Anmeldenummer: PCT/EP2018/051868
(87) Internationale Veröffentlichungsnummer: WO 2018/138214

(56) Entgegenhaltungen:
- EP-A1- 2 896 378
- EP-A1- 2 932 929
- EP-A2- 1 055 828
- WO-A1-2014/035764
- DE-A1-102013 105 744
- DE-A1-102014 108 225
- DE-A1-102014 109 200
- FR-A1- 3 021 571
- US-A1- 2015 223 844

## Beschreibung

### Hintergrund der Erfindung

Die vorliegende Erfindung betrifft ein Implantationsset zum Implantieren einer medizinischen (Knochen-)Schraube in einen menschlichen oder tierischen Körper enthaltend ein Werkzeug und eine zum Aufnehmen des Werkzeugs ausgelegte Schraube. Des Weiteren betrifft die vorliegende Erfindung eine Schraube sowie ein entsprechend geformtes Werkzeug, die zur Ausbildung des erfindungsgemäßen Implantationssets vorgesehen und ausgebildet sind. Die Schraube bzw. deren Schraubenkopf weist einen Werkzeugaufnahme-/einführbereich, genauer einen Einführschrägenbereich auf, der proximal zu einem Drehmoment-Aufnahme-/Einleitabschnitt im Schraubenkopf ausgeformt ist. Der Einführschrägenbereich ist in Form- und Kraftschluss mit einer Zentrierfläche des Werkzeugs. Die Zentrierfläche des Werkzeugs ist proximal zu einen Drehmoment-Übertragungsabschnitt des Werkzeugs ausgebildet, der dafür konfiguriert und angepasst ist, in den Drehmoment-Aufnahme-/Einleitabschnitt im Schraubenkopf eingeführt zu werden.

### Stand der Technik

Aus dem Stand der Technik sind bereits Schrauben zum Implantieren in menschliche und tierische Körper bekannt, wie zum Beispiel Pedikelschrauben. Auch sind passende Werkzeuge bekannt, die in ein Sackloch mit Vielnutprofil (Vielrundprofil) in einem Schraubenkopf einer solchen Schraube eingesetzt oder auf diesen aufgesetzt werden können, um ein Drehmoment auf die Schraube aufzubringen.

Formschlüssige Verbindungen von Schraube und Werkzeug zur Kraftübertragung sind herkömmlicherweise nach Art einer Kreuzschlitz-, Inbus- oder Torx-Ausprägung gestaltet. Solche Kreuzschlitz-, Inbus- oder Torx-Gestaltungen der Koppelabschnitte der beiden Bauteile sind weit verbreitet und wohl bekannt. Unter "Torx" wird eine solche Schraubenart verstanden, die eine vielrundartige Mitnahmeprofilausgestaltung aufweist. Sie ähnelt einem Davidstern mit abgerundeten Spitzen und Nuten sowie abgerundeter Ecken. Der Werkzeugeingriff zwischen Werkzeug und Schraube kann hohe Drehmomente auch ohne Beschädigung des Schraubenkopfes übertragen. Unter einem solchen Torx wird auch beispielsweise ein "Sechsrund" verstanden.

Es ist bemerkenswert, dass bei solchen Torx-Profilen das Auf-/Einsetzen des Werkzeugs erleichtert ist und eine verbesserte Kraftübertragung, im Vergleich zu Schlitz- oder Kreuzschlitzschrauben möglich ist. Wegen der bei einem Vielnutprofil bzw. Torx-Profil senkrecht verlaufenden Antriebs-/Drehmomentübertragungsflächen ist keine oder nur eine kaum erhöhte Andrückkraft in Werkzeug-Achsrichtung beim Festziehen erforderlich, und es treten keine oder kaum Rückschubkräfte auf, wodurch das sog. "Cam-out" verhindert wird. Auch ist bemerkenswert, dass Drehkräfte nicht, wie etwa bei Inbus-Systemen üblich, punktuell an den Ecken angreifen, sondern mittig und flächig an den Flanken der einzelnen die Antriebsflächen ausbildenden (in Achsrichtung des Werkzeugs sich erstreckender) Stege. Die Folge ist, dass das aus einem männlichen und weiblichen Element bestehende System deutlich verschleißärmer ist. Insbesondere in der Chirurgie und beim Zahnersatz bietet sich daher der Einsatz von Schrauben mit einer entsprechenden Werkzeugaufnahme und ein daran angepasstes Werkzeug an. Dadurch können Knochenbruchbehandlungen präziser und problemloser durchgeführt werden. Auch können Implantate einfacher gesetzt werden.

Unter "Torx" werden hier sowohl Innentorx- als auch Außentorx-Profilarten verstanden. Es werden dabei T-, E- als auch TR-Varianten umfasst, genauso wie Torx+- als auch HexStix-Varianten.

In nahezu allen Bereichen werden solche Verbindungen, die auf Formschluss setzen, verwendet, insbesondere auch in der Chirurgie. Im Bereich der Wirbelsäulenchirurgie werden solche Verbindungen eingesetzt, bspw. um Knochenschrauben einzuschrauben, Sicherungsschrauben anzuziehen oder ähnliche bewegliche Teile zu sichern. Während bisher der Inbus die erste Wahl war, setzt sich aber aus den genannten Gründen, zunehmend die Torx-Ausgestaltung durch.

Im medizinischen Einsatzgebiet zeigen sich insbesondere die Vorteile von Inbus- und Torx-Geometrien, bei denen der zylindrische Anteil überwiegt, so dass der Schraubendreher während des Einschraubens nicht zusätzlich axial in den Schraubenkopf gedrückt werden muss, wie dies bei Kreuzschlitz-Geometrien der Fall ist, sondern lediglich der Einschraubtiefe kraftfrei folgen muss.

Im Bereich der Wirbelsäule verwendete Schrauben, z.B. Pedikelschrauben, müssen oft "in situ", in der Tiefe oder ohne Sichtkontakt angekoppelt werden. Unter anderem ist das insbesondere bei dem als "S4"- und "Ennovate"-Schraubendrehern der Fall, bei denen die Knochenschraube bzw. deren Schraubenkopf von einer sogenannten "Tulpe" umschlossen wird, die die Sicht behindert, wodurch das Werkzeug nur nach Gefühl angekoppelt bzw. in die schraubenkopfseitige Werkzeugaufnahme eingeführt werden muss. Die taktile Rückkoppelung ist daher für einen Einsetzerfolg (erfolgreiches Einsetzen des Werkzeugs in oder auf die Schraube) maßgeblich.

Knochenzement findet unter anderem Anwendung bei Patienten deren Knochenqualität nicht ausreichend ist, um darin eine Schraube fest zu verankern. Hierfür wird in einen Wirbel der Wirbelsäule ein Loch gebohrt, das mit Knochenzement oder einer anderen stabilisierenden Substanz gefüllt wird und anschließend wird eine Pedikelschraube eingebracht. Knochenzement ist dabei meist ein zwei Komponenten-Klebstoff, der unter anderem zu Plexiglas aushärten kann.

Die DE 10 2013 104 744 A1, nach Haas et al., offenbart eine Implantationsschraube und ein Werkzeug eines Implantationssets mit Relativrotationserzwingung. Genauer wird ein Implantationsset offenbart, bei dem die Schraube einen Werkzeugaufnahme-/Drehmomentübertragungsbereich aufweist, an den sich zumindest ein Schrägenbereich zum (Ein-)Führen des Werkzeugs in den Werkzeugaufnahme-/Drehmomentübertragungsbereich (während des Einsetzens/Einführens) in die Schraube anschließt.

Die WO 2011/043799 A1, nach Lange et al, offenbart ein Fixiersystem und einen Schraubendreher für die Verwendung mit demselben.

Die im Bereich der Wirbelsäule verwendeten Schrauben, z. B. Pedikelschrauben, werden mittels eines Schraubendrehers in die Wirbelsäule eingeschraubt. Die Verbindungsstelle zwischen Pedikelschraube und Schraubendreher ist im Stand der Technik meist nur durch eine vielrundartige Mitnahmeprofilausgestaltung gemäß vorstehender Torx-Geometrie gegeben, genauer gesagt durch deren senkrecht/parallel zur Werkzeugachse verlaufenden Antriebsflächen. Eine nicht achsgenaue Verbindung zwischen Schraube und Schraubendreher kann dazu führen, dass es durch das Ein- oder Ausschrauben der Knochenschraube zur Auslockerung oder im schlimmsten Fall zum Bruch eines Pedikels kommen kann. Auch besteht das Problem, dass beim Aufsetzen/Einführen des Schraubendrehers die Dichtigkeit gegenüber Knochenzement sowie die Torsionsfestigkeit nicht zuverlässig gesichert werden. Des Weiteren wird die Ausrichtung zwischen Schraubendreher und Schraube bei einem wirkenden Drehmoment nicht (ausreichend) stabilisiert, wodurch die Ausrichtung der Schraubendreherachse zur Achse der Schraube instabil ist. Ein Wiederaufsetzen/Wiedereinführen des Werkzeugs in die Schraube gestaltet sich auch oft als schwierig insbesondere dann, wenn die Schrauben bereits implantiert ist. Ähnliche Werkzeuge und Schrauben sind in WO2014/035764 A1 und EP2896378 A1 offenbart.

### Kurzbeschreibung der Erfindung

Es ist daher die Aufgabe der Erfindung, eine (feste) Verbindung zwischen Schraubendreher und Schraube (Pedikelschraube) bereitzustellen, die ein möglichst hohes Drehmoment übertragen kann. Eine weitere vorteilhafte Aufgabe der Erfindung ist es, dass die Schraube kein Spiel zu dem Schraubendreher hat und achsgenau zur Achse des Schraubendrehers ausgerichtet werden kann, wobei die Achsgenauigkeit selbst unter Einwirkung größerer Hebelkräfte bestehen bleibt. Eine weitere Aufgabe der Erfindung ist es, dass bei einem Wiederankoppeln des Schraubendrehers an der Schraube, vor allem im intraoperativen (insitu) Bereich, die Achsgenauigkeit zwischen der Schraube und dem Schraubendreher, durch das feste Verbinden der Teile, wieder hergestellt werden kann. In anderen Worten muss es zu einer Ausrichtung der Achse des Schraubendrehers zu der Achse der Schraube (zwangsläufig) führen, wenn der Schraubendreher mit der Schraube verbunden/gekoppelt wird. Des Weiteren soll verhindert werden, dass Knochenzement unkontrolliert austreten kann.

Diese Aufgabe wird durch ein Implantationsset mit den Merkmalen des Anspruchs 1 und durch ein medizinisches Werkzeug mit den Merkmalen des Anspruchs 6 gelöst.

Des Weiteren wird die Aufgabe durch eine Schraube, vorzugsweise Knochenschraube, weiter vorzugsweise Pedikelschraube und ein hierzu passendes Einschraub-Werkzeug gelöst, die sämtlich unabhängig beanspruchbar sind.

Der Grundgedanke der vorliegenden Erfindung besteht demzufolge darin, eine Schraube, vorzugsweise Knochenschraube, weiter vorzugsweise Pedikelschraube in deren Schraubenkopfbereich mit einer profilierten, sackloch-artigen Werkzeugaufnahme auszubilden, mit einem (distalen) Drehmoment-Einleitabschnitt, vorzugsweise in Torx-/Vielnut-/Inbusform, dessen (proximaler) äußerer Umfangsrand/Umfangskante zu einem Einführschrägenbereich geformt ist. Dieser Einführschrägenbereich stellt/bildet erfindungsgemäß eine Fortführung des Drehmoment-Einleitabschnitts, vorzugsweise in Torx-/Vielnut-/Inbusform dar, wobei die dabei sich ausbildenden Stege und Nuten im (proximal an den Drehmoment-Einleitabschnitt unmittelbar sich anschließenden) Einführschrägenbereich nicht achsparallel wie im Drehmoment-Einleitabschnitt sondern unter einen Winkel größer 0° zu der (Schrauben-)Achse in Richtung proximal schräg nach außen in Verlängerung zu den Axialstegen und Axialnuten im Drehmoment-Einleitabschnitt verlaufen. Die Nuten/Einbuchtungen im Einführschrägenbereich sind dabei schalen- oder kugelkalottenförmig ausgebildet und bilden in Draufsicht auf die Werkzeugaufnahme jeweils trichter- bzw. sichelförmige Konturen, die demzufolge bevorzugt auf einer Kreisbahn um die Sacklochachse in vorzugsweise gleichem Umfangsabstand angeordnet sind.

Der Außenradius der Nuten/Einbuchtungen im Einführschrägenbereich ist so bemessen, dass sich die Außenradien jeweils zweier, in Umfangsrichtung benachbarter Nuten/Einbuchtungen im Einführungsschrägenbereich schneiden.

Das zugehörige/passende (Einschraub-)Werkzeug hat einen Schaft, an dessen distalem Endbereich ein Drehmomentübertragungsabschnitt vorzugsweise in Torx-/Vielnut-/Inbusform ausgebildet oder angeordnet/anordenbar ist und der dafür konfiguriert und/oder angepasst ist, in den schraubenseitigen Drehmoment-Einleitabschnitt (formschlüssig für eine Drehmomentübertragung) eingesteckt zu werden. Der Drehmomentübertragungsabschnitt des Werkzeugs kann bevorzugt an seinem distalen Ende kegelstumpfartig angeschrägt sein (Anfasung), derart, dass die Werkzeugspitze bzw. die dort sich ausbildenden kegelstumpfartig sich zuspitzenden/abflachenden, stegförmigen Vorsprünge des Torx-/Vielnut-/Inbusprofils durch gleitendes In-Eingriff-Kommen mit dem schraubenseitigen Einführschrägenbereich bzw. den dort ausgebildeten (Kugel-kalottenförmigen) Nuten/Einbuchtungen in der sackloch-artigen Werkzeugaufnahme zentriert werden und der Werkzeugschaft koaxial zur Schraubenachse (Sacklochachse) ausgerichtet wird. Da ein gleitendes Einführen des werkzeugseitigen Drehmomentübertragungsabschnitt in den schraubenseitigen Drehmoment-Einleitabschnitts innerhalb der sackloch-artigen Werkzeugaufnahme gewährleistet sein muss, ergibt sich zwischen den Nuten und Stegen in der Werkzeugaufnahme und den Stegen und Nuten am Werkzeug (-Schaft) zwangsläufig ein Spiel, das eine Kipptendenz des Werkzeugschafts in der sackloch-artigen Werkzeugaufnahme verursachen kann.

Aus diesem Grund bildet der Werkzeugschaft am proximalen (hinteren) Ende seines Drehmomentübertragungsabschnitts einen radial vorragenden Wellen-/Schaftabsatz, in den das Profil des schraubenseitigen Einführschrägenbereichs spiegelbildlich/komplementär eingearbeitet ist. D.h., im werkzeugseitigen Wellenabsatz sind eine Anzahl (axial) in Richtung distal sich erstreckender, kugelförmiger Vorsprünge, entsprechend (passend zu) den kugelkalottenförmigen Rücksprüngen/Einbuchtungen im schraubenseitigen Einführschrägenbereich ausgebildet, die erfindungsgemäß bei/mit vollständigem Einführen des werkzeugseitigen Drehmomentübertragungsabschnitts in den in den schraubenseitigen Drehmoment-Einleitbereich/-abschnitt in die kugelkalottenförmigen Rücksprünge/Einbuchtungen im schraubenseitigen Einführschrägenbereich eingreifen/eingedrückt werden und dadurch den Werkzeugschaft in der sackloch-artigen Werkzeugaufnahme der Schraube radial einspannen. Zudem ergibt sich ein Formschluss in Umfangsrichtung. Auf diese Weise kann die vorstehend beschriebene Kipptendenz verringert bzw. vermieden werden.

In anderen Worten ist der Bereich der Schraube, der zur Aufnahme eines/des Schraubendrehers vorgesehen und angepasst ist, also der Bereich der Schraube, der eine relative Vertiefung (Sackloch) am Schraubenkopf aufweist, passgenau zu einem korrespondierenden Schraubendreherkopf ausgebildet. Demnach wird in der vorliegenden Erfindung ein Schraubendreher beschrieben, welcher im Zusammenspiel beispielsweise mit mono- oder polyaxialen Pedikelschrauben eine achsgenaue und torsionsfeste, sowie zementdichte, Verbindungsstelle herstellt, wobei der schraubenseitige Einführschrägenbereich im Wesentlichen dichtend mit dem entsprechend geformten werkzeugseitige Wellenabsatz in Anlage kommt. Bedingt durch diese spezielle Ausgestaltung ist auch ein insitu Wiederankoppeln in einfacher und sicherer Weise möglich.

Die vorliegende Erfindung betrifft somit ein Implantationsset zum Implantieren einer Schraube in einen menschlichen oder tierischen Körper, aufweisend ein (Einschraub-) Werkzeug und eine zum Aufnehmen des Werkzeugs ausgelegte Schraube, wofür die Schraube einen Werkzeugaufnahmebereich/-abschnitt in ihrem Schraubenkopf aufweist, in den (oder auf den) ein Drehmomentübertragungsbereich/-abschnitt des Werkzeugs zur Drehmomentübertragung einsetzbar/aufsetzbar oder eingesetzt/aufgesetzt ist, wobei sich an dem Werkzeugaufnahmebereich an dessen proximalem Rand (Einführöffnung) zumindest ein Einführschrägenbereich/-abschnitt u.a. zum Führen/Ausrichten des Werkzeugs (Werkzeugschafts) während des Einsetzens in die Schraube ausbildet. Erfindungsgemäß bildet das Werkzeug zumindest eine (ringförmige) Zentrierfläche (Wellen-/Schaftabsatz) aus, die den Drehmomentübertragungsbereich proximal (entfernt zur Werkzeugspitze) begrenzt und mit dem/auf den Einführschrägenbereich so abgestimmt ist, dass beim in Kontakt gelangen (Aufsitzen) der Zentrierfläche mit (auf) dem Einführschrägenbereich ein Form- und Kraftschlusszwischen Einführschrägenbereich und Zentriefläche zusätzlich zum Drehmomentübertragungsbereich/-abschnitt entsteht.

Die Fähigkeit (Wirkung) zur Übertragung des Torsionsmoments zwischen dem Schraubendreher bzw. dem Schraubendreherschaft und der Schraube bzw. der Knochenschraube wird somit durch den Form- und Kraftschluss zwischen der Zentrierfläche und dem Einfuhrschrägenbereich zusätzlich verstärkt (erhöht). Bei form- und(oder kraftschlüssiger Anlage der stirnseitigen Senkung (Einführschrägenbereich) der Schraube mit der dazu komplementären Zentrierfläche (Wellen-/Schaftabsatz mit radial und axial vorragenden, semi-kugelförmigen Vorsprüngen) am Schraubendreher erfolgt die Torsionsübertragung über einen gegenüber dem darunter/distal liegenden Vielrund (Vielnutprofil) vergrößertem Querschnitt/Durchmesser. Hierdurch wird eine höhere Torsionsfestigkeit erreicht. Durch die komplementäre Passung zwischen der werkzeugseitigen Zentrierfläche und der schraubenseitigen Senkung bzw. dem Einführungsschrägenbereich wird eine höhere Abdichtung bzw. Dichtigkeit zur sicheren Applikation von Knochenzement erreicht.

Die erfindungsgemäße Schraube ist bevorzugt eine Pedikelschraube mit
- einer, einen Schraubenschaft und den vorstehend definierten Schraubenkopf aufweisenden Knochenschraube,
- einem Body bzw. Tulpe, der/die beweglich am Schraubenkopf gehalten ist und ein Innengewinde, vorzugsweise zur Aufnahme einer Madenschraube, aufweist (entspricht einer polyaxialen Pedikelschraube) sowie optional
- einem Insert (Einlage zur Verspannung von Schraubenkopf und Tulpe) und der Madenschraube.

Das erfindungsgemäße Einschraub-Werkzeug hat bevorzugt
- einen Werkzeugschaft mit einem distalen Drehmomentübertragungabschnit sowie einem proximal hierzu angeordneten Wellen-/Schaftabsatz (Zentriefläche) entsprechend der vorstehenden Definitionen,
- einen fest mit dem Werkzeugschaft verbundenen/verbindbaren Gegenhalter in Form eines vorzugsweise im Querschnitt elliptischen Wellen-Schaftabsatzes/Schaftrings proximal zu der Zentrierfläche, der dafür konfiguriert und/oder angepasst ist, in einen Längspalt der Tulpe eingeführt zu werden, um bei einem rotatorischen Festhalten des Werkzeugschafts die Tulpe ebenfalls rotatorisch festzuhalten und
- ein hülsenförmiges Gewindeteil mit axialer Durchgangsbohrung, in welche der Werkzeugschaft drehbar sowie axialbeweglich eingeführt ist, derart, dass sich das hülsenförmige Gewindeteil proximal zum Gegenhalter anordnet, wobei das hülsenförmige Gewindeteil an seinem distalen Endabschnitt ein Außengewinde hat, das dafür konfiguriert und/oder angepasst ist, mit dem Innengewinde des Body/Tulpe in Eingriff zu kommen und das hülsenförmige Gewindeteil an seinem proximalen Endabschnitt vorzugsweise ein Eingriffsprofil aufweist, das mit einem Drehmoment-Beaufschlagungswerkzeug/-mittel z.B. einem Schraubenschlüssel in Eingriff bringbar ist, um das hülsenförmigen Gewindeteil in den Body/Tulpe (temporär) einzudrehen, während der Body/Tulpe mittels des Werkzeugschafts und Gegenhalters rotatorisch festgehalten wird.

Vorteilhafte Ausführungsformen sind nachstehend beschrieben und werden in den weiteren Unteransprüchen beansprucht.

In einer vorteilhaften Ausführungsform sind an einer zylindrischen Seitenfläche des Werkzeugs Drehmomentübertragungsbereiche, genauer axial sich erstreckende Rippen oder Stege, ausgebildet, die sich über einen distal sich daran anschließenden, kegelstumpfförmigen Seitenflächenabschnitt bis zu einer Spitze des Werkzeugs erstrecken. In anderen Worten ausgedrückt, weist die kegelstumpfförmige Seitenfläche bzw. der konusförmige Spitzenabschnitt des Schraubendrehers ein kegelstumpfförmiges bzw. konusförmiges Vielrund auf. Dieses bewirkt, dass beim Anziehen des Gewindeteils der konusförmige Spitzenabschnitt (die Schraubendreherspitze) nicht auf der proximalen Stirnseite der Schraube, also an der Seite, an der sich die Senkung/das Sackloch an dem Schraubenkopf befindet, stumpf aufsitzen kann, sondern im Zusammenspiel mit der stirnseitigen Senkung bzw. deren Einführschrägenbereich an der Schraube automatisch so ausgerichtet wird, dass die Vielrundgeometrie des Schraubendreherschafts sich zu der Vielrundgeometrie der Schraube ausrichtet.

Wie vorstehend bereits ausgeführt wurde, weist das Werkzeug gemäß einer bevorzugten Weiterbildung den Schraubendreherschaft auf, an dem das Gewindeteil angebracht/gelagert ist, das ein Außengewinde an seinem distalen Ende aufweist, das in das Innengewinde des relativ zum Schraubenschaft/Schraubenkopf beweglichen Body (Tulpe) eindrehbar ist, der an der Schraube angeordnet ist. Das (hülsenförmige) Gewindeteil ist dabei auf dem Schaft des Schraubendrehers oberhalb des Gegenhalters angeordnet und umschließt mit seiner zylindrischen Hohlform den Schraubendreher. Das Außengewinde befindet sich auf der der Schraube und somit auch dem Gegenhalter zugewandten Seite des Gewindeteils. Durch ein Anziehen des Gewindeteils kann eine achsparallele Ausrichtung des Schraubendrehers zur Schraube erzwungen werden. In anderen Worten ausgedrückt, wird die Schraubenlängsachse und die Schraubendreherlängsachse angepasst und sind somit zueinander parallel (coaxial).

In einer weiteren vorteilhaften Ausführungsform weist der Schraubendreher an seinem Schaft den Gegenhalter auf, der in dem Body aufgenommen werden kann. Der Gegenhalter ermöglicht durch eine Aufnahme in dem Body und seiner Form, dass der Body sich beim Eindrehen des Gewindeteils nicht mitdrehen kann. Der Gegenhalter ist also in seiner Form vorgesehen und angepasst, von dem Body aufgenommen zu werden. Dadurch, dass der Body an zwei diametral gegenüberliegenden Seiten Öffnungen/Längsschlitze an seinen Seiten aufweist, ist es auch möglich, den Schraubendreherschaft schräg zur Schraubenachse in den Body einzusetzen. Dieser zusätzliche Freiheitsgrad stellt beim Wiederankoppeln des Schraubendrehers eine erhebliche Vereinfachung dar.

In einer weiteren vorteilhaften Ausführungsform weist der Body das Insert auf, bzw. nimmt dieses auf, wobei der Body eine Innenbohrung ausbildet, die vorgesehen und angepasst ist zur Aufnahme des Schraubendreherschafts, genauer zur Aufnahme des Schaftes des Schraubendrehers unterhalb des Gegenhalters. Dabei weist der Body innenseitig eine schalenartige Form auf der dem Werkzeug zugewandten Seite auf, damit beim Einführen des Werkzeugs die Werkzeugspitze in Richtung der Innenbohrung geführt wird.

In einer weiteren vorteilhaften Ausführungsform ist die erfindungsgemäße Konfiguration aus Schraube, Body, Schraubendreher, ggf. Insert und Gewindeteil so ausgestaltet, dass das Gewindeteil erst dann in das Innengewinde des Bodys eingreifen kann, wenn die Schraubendreherspitze so ausgerichtet ist, dass sich die Vielrundgeometrie des Schraubendrehers und der Schraube zueinander ausgerichtet haben (d.h. der Drehmomentübertragungsabschnitt des Werkzeugs bereit in den Drehmomenteinleitabschnitt der Schraube eingeführt ist. Dadurch wird eine fehlerhafte Ankopplung des Schraubendrehers in einer nicht zur Schraubenachse ausgerichteten Weise ausgeschlossen.

Durch das vorstehend beschriebene Implantationsset wird das insitu Wiederaufsetzen des Schraubendrehers auf die Schraube durch ein aktives Verspannen des Gewindeteils mit dem Body, so dass der Schraubendreher und die Schraube zueinander verspannt werden, ermöglicht. Dadurch wird ein Ausrichten des Schraubendrehers mit der Schraube herbeigeführt. Der Schraubendreher kann auch dann aufgesetzt werden, wenn der Schraubendreher nicht aktiv in Achse mit dem Body gebracht wird. Die Ausrichtung der Bodyachse zur Schraubendreherachse erfolgt automatisch beim Aufsetzen des Schraubendrehers. Durch diese automatische Ausrichtung wird das Aufsetzen im Allgemeinen, insbesondere aber das Wiederaufsetzen des Werkzeugs auf die Schraube vereinfacht, da intraoperativ eine aktive/nicht automatische Ausrichtung der Schraubendreherachse zur Bodyachse erschwert sein kann.

Die Erfindung stellt ein einfaches und sicheres Wiederankoppeln des Schraubendrehers an die Schraube, vorzugsweise an eine Pedikelschraube sicher. Das Ausrichten des Schraubendrehers zu der Schraube wird ermöglicht, ohne die Notwendigkeit, den Schraubendreher gleich zu Beginn des Wiederankopplungsvorgangs auf die Achse des Bodys ausrichten zu müssen.

Bei einem insitu Wiederankoppeln wird die Schraubendreherspitze in die Pedikelschraube/deren Sackloch eingeführt. Die konusförmige Spitze zentriert sich dabei über der Bohrung des Inserts. Der Gegenhalter des Werkzeugs muss dabei so ausgerichtet werden, dass er in zumindest eine Stabaufnahmenut/Längsschlitz des Bodys gebracht werden kann. Das Außengewinde des hülsenartigen Gewindeteils kann bei einem schrägen Einführen des Werkzeugs noch nicht in Eingriff mit dem Innengewinde des Bodys gebracht werden. Der Schraubendreher wird nun in die Pedikelschraube vorgeschoben/eingeführt. Durch die Zentrierspitze des Werkzeugs und der Passung zwischen dem Schaftabschnitt/Drehmomentübertragungsabschnitt unterhalb/distal zum Gegenhalter und der Innenbohrung des Inserts richtet sich der Body zum Schraubendreher aus. Wenn die Werkzeuggeometrie des Schraubendrehers zur Werkzeugaufnahmegeometrie der Schraube in Rotationsrichtung ausgerichtet ist, ist auch die Achse des Bodys zur Achse des Gewindeteils so weit ausgerichtet, dass das Gewindeteil in das Innengewinde des Body eingeschraubt werden kann. Der distale Konusabschnitt des Werkzeugs ist komplementär zu den kugel-kalottenförmigen Nuten/Senkungen/Einbuchtungen im Einführschrägenbereich der Schraube, so dass diese zusätzlich zu einer einfachen Ausrichtung der Werkzeuggeometrie führt. Beim Anziehen des Gewindeteils im Body/Tulpe wird eine feste Ausrichtung der Achsen erreicht, indem die kugelförmigen Vorsprünge am Werkzeugschaft-seitigen Schaftabsatz in die kugel-kalottenförmigen Nuten/Senkungen/Einbuchtungen im Einführschrägenbereich der Schraube eingedrückt werden. Dadurch ergibt sich ein Form- und Kraftschluss der kugelförmigen Vorsprünge mit den kugel-kalottenförmigen Nuten/Senkungen/Einbuchtungen. Auch wird so eine zementdichte Verbindung zwischen dem Schraubendreher und der Schraube erreicht.

Zusammenfassend ergibt sich eine Vielzahl von Vorteilen durch die erfindungsgemäße Ausgestaltung des Implantationssets. Es wird eine sehr stabile Verbindung zwischen Schraube und Schraubendreher erreicht. Die Achse des Schraubendrehers wird zuverlässig mit der Achse der Schraube ausgerichtet. Das insitu Wiederankoppeln des Schraubendrehers an die Schraube wird wesentlich vereinfacht. Durch eine Form- und Kraftschlussverbindung wird eine hohe Drehmomentfestigkeit der Verbindungsstelle erreicht. Die Verbindungsstelle zwischen Schraubendreher und Schraube ist dicht genug, um Leckagen bei der Applikation von Knochenzement zu vermeiden. Der Effekt der Ausrichtung der Achse des Schraubendrehers zur Achse der Schraube wird unter Einwirkung des Einschraubmoments verstärkt. Die vorliegende Verbindung bietet zusätzlich eine kostengünstige Konstruktion durch die Gesamtbearbeitbarkeit des Arbeitsendes durch nur ein Werkzeug wie zum Beispiel eine Gesenkfräse mit einem Durchmesser von vorzugsweise 0,8 mm.

### Figurenbeschreibung

Die Erfindung wird nachfolgend anhand eines bevorzugten Ausführungsbeispiels unter Bezugnahme auf die begleitenden Figuren näher erläutert. Es zeigen:
Fig. 1 die Komponenten eines erfindungsgemäßen Implantationssets in Eingriff zueinander.
Fig. 2 einen Werkzeugkopf eines Werkzeugs gemäß der vorliegenden Erfindung
Fig. 3 ein Gewindeteil angeordnet an dem Werkzeug vorliegend in Form eines Schraubendrehers.
Fig. 4 ein Body/eine Tulpe angebracht an einer Schraube vorliegend einer Pedikelschraube.
Fig. 5 ein Insert angeordnet in dem Body/der Tulpe.
Fig. 6 einen Schraubenkopf der zu implantierenden Schraube/Pedikelschraube vorliegend der polyaxialen Bauart.
Fig. 7 den Schraubenkopf aus Fig. 6 in einer detaillierten Ansicht.
Fig. 8 eine Seitenansicht des Werkzeugs im verspannten Zustand in der Schraube, das in dem Insert und dem Body angeordnet ist.

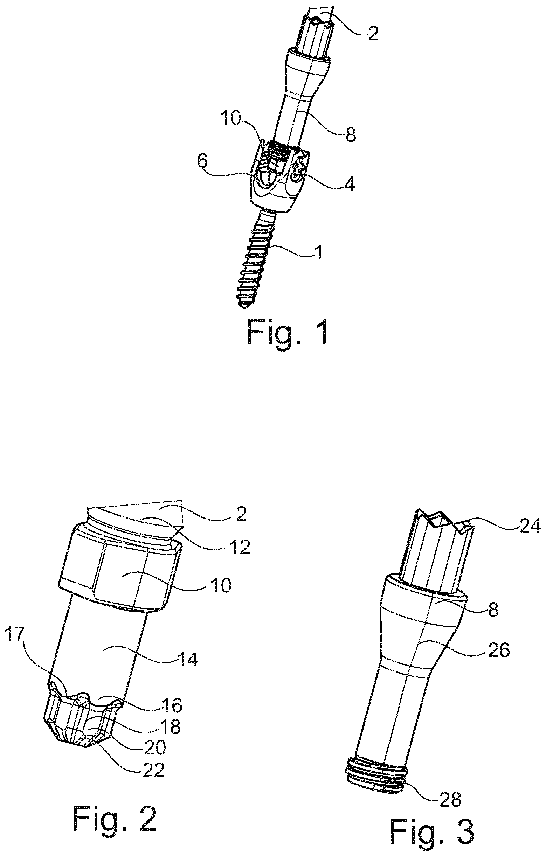

In Fig. 1 sind die Komponenten eines Implantationssets gemäß dem bevorzugten Ausführungsbeispiel der vorliegenden Erfindung in Eingriff zueinander gezeigt. Eine Schraube, vorliegend Pedikelschraube 1 steht demnach in Eingriff mit einem (Einschraub-)Werkzeug/Schraubendreher 2. Axial über die Schraube 1 ist an deren proximalem Schraubenkopf ein beweglicher Body (Tulpe) 4 gestülpt, der optional ein Insert/Inlay 6 aufweist, das relativ axialbeweglich zum Body 4 gehalten ist.

Radial über dem Werkzeug 2 bzw. dessen Werkzeugschaft 12 ist ein Gewindeteil/Gewindehülse 8 von der proximalen Seite in Richtung hin zu der distalen Werkzeugspitze/Schaftspitze des Werkzeugs 2 gestülpt, das auf einem Gegenhalter (in etwa ovaler/elliptischer Schaftring) 10 axial aufliegt, der am Schraubendreher/Werkzeugschaft an einer bestimmten/bestimmbaren Axialposition (axialfest) angeordnet/ausgebildet ist.

Fig. 2 zeigt den Kopf/Spitzenabschnitt/Werkzeugkopf des Werkzeugs 2 in detaillierter Darstellung.

Der Werkzeugkopf ist an dem vorzugsweise zylindrischen Schraubendreherschaft 12 ausgebildet, der von einem Griff (nicht gezeigt) kommend zu dem Gegenhalter (10) führt. Der Gegenhalter 10 ist vorzugsweise im Querschnitt hammerförmig (oval/elliptisch) oder in Form einer Flügelmutter ausgestaltet, wobei zwei diametral gegenüberliegende Seiten/Flügel radial über den Umfang des oberhalb (proximal) des Gegenhalters (10) sich erstreckenden zylindrischen Schafts 12 ragen. Ein den Werkzeugschaft 12 in Richtung distal verlängernder Schaftabschnitt 14 unterhalb/distal des Gegenhalters 10 ist im Durchmesser kleiner als der Schaft 12 oberhalb (proximal) des Gegenhalters 10. Der Schaftabschnitt 14 unterhalb (distal) des Gegenhalters 10 geht distal in einen Zentrierflächenabschnitt bzw. Wellen-/Schaftabsatz 16 für das Zentrieren des Werkzeugschafts 14 bzgl. der Schraube 1, für die zusätzliche Torsionsübertragung und für das Abdichten über. Der Zentrierflächenabschnitt 16 geht distal in ein Vielrund(-profil)/Drehmomentübertragungsabschnitt 18 über, der axial sich erstreckende, radial vorragende sowie in Umfangsrichtung beabstandete Rippen/Stege/Zähne 20 an seiner zylindrischen Außenfläche aufweist. Der Übergang zwischen Zentrierflächenabschnitt 16 und Vielrund 18 ist dabei vorzugsweise fließend durch abgerundete in Umfangsrichtung aneinander gereihte Geometrien (Zentrierflächen/Vorsprünge 17) ausgestalten.

In anderen Worten ausgedrückt sind am Wellen-/Schaftabsatz 16 eine Anzahl in Umfangsrichtung beabstandeter Vorsprünge 17 ausgebildet, die sich jeweils axial wie auch radial erstrecken und zwischen sich in Umfangsrichtung beabstandete Nuten/Vertiefungen in axial- und Radialrichtung bilden. Die Vorsprünge 17 und Nuten am Wellen-/Schaftabsatz 16 sind dabei in axialer Verlängerung zur den Rippen/Stegen 20 und Nuten im Drehmomentübertragungsabschnitt 18 angeordnet.

Die Umfangsfläche des kegelstumpfförmigen Werkzeugsendabschnitts 22, bzw. Zentrierspitze, bildet ebenfalls eine Vielrundgeometrie (in Verlängerung zu der Vielrundgeometrie im Drehmomentübertragungsabschnitt 18 aus. Der hauptsächliche Drehmomentübertragungsbereich/-abschnitt 18 wird durch die (axial sich erstreckenden) Rippen 20 des Vielrunds ausgebildet. Die Rippen 20 verlaufen demnach von der zylindrischen Umfangsfläche des Vielrunds über die Umfangsfläche des sich distal an den Drehmomentübertragungsbereich 18 anschließenden Schaft-Kegelstumpfes 22 hin zu der stirnseitig abgeflachten Werkzeugschaftspitze.

Aus der Fig. 2 ist ferner zu erkennen, dass sich die Zentrierflächen/Vorsprünge im Bereich des Wellen-/Schaftabsatzes 16 und die Rippen 20 axial zueinander (in Verlängerung) ausrichten, wobei die einzelnen Zentrierflächen/Vorsprünge 17 im Wellen-/Schaftabsatz 16 jeweils als semi-kegelförmige axial-radiale Vorsprünge über den Rippen 20 ausgebildet sind und durch im Wellen-/Schaftabsatz 16 sich ausbildende schalen-/kalottenförmige Einbuchtungen/Nuten in Umfangsrichtung beabstandet sind, die sich ebenfalls in Verlängerung zu den Nuten im Drehmomentübertragungsabschnitt 18 anordnen.

Fig. 3 zeigt das hülsenförmige Gewindeteil 8 in Detaildarstellung, das an dem erfindungsgemäßen Schraubendreher 2, d.h. am Werkzeugschaft 12 relativ bewegbar angeordnet werden kann/ist. Das Gewindeteil 8 weist ein durchgehendes Loch (Axialdurchgangsbohrung) 24 zur dreh- und axialverschiebbaren Aufnahme des Schraubendreherschafts 12 auf, wobei der Lochdurchmesser komplementär zum Außendurchmesser des Schafts 12 oberhalb des Gegenhalters 10 für ein im Wesentlichen spielfreies Lagern ist. Das Gewindeteil 8 liegt axial auf dem Gegenhalter 10 des Werkzeugs 2 auf. An der dem Gegenhalter 10 zugewandten axialen Endseite des Gewindeteils 8, d.h. dem distalen Ende des Gewindeteils 8, ist daran unterhalb eines bevorzugt außen glatten Hülsenabschnitts 26 ein Außengewinde 28 ausgebildet. Darüber hinaus ist an dem oberen, proximalen Ende des Gewindeteils 8 ein axiales Zackenprofil ausgearbeitet, um über ein entsprechendes Werkzeug (z.B. nicht gezeigter Schraubenschlüssel) ein Drehmoment auf das Gewindeteil 8 aufbringen zu können.

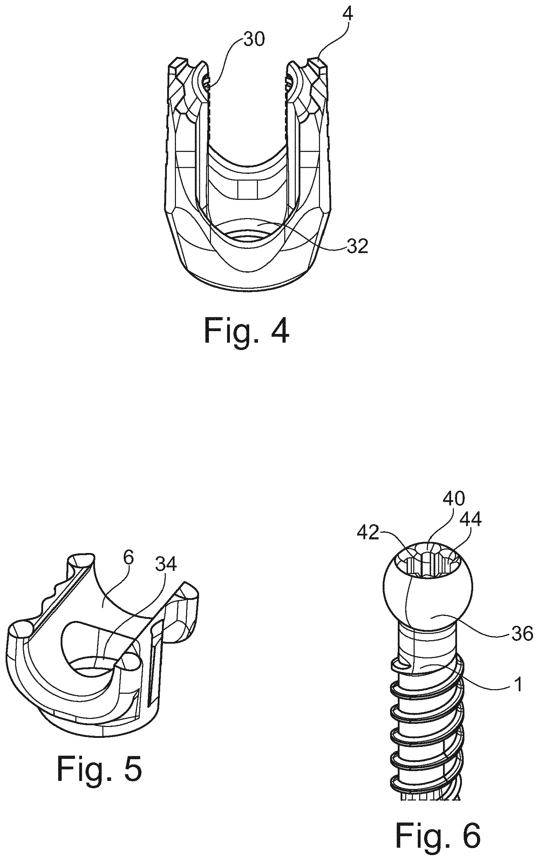

Fig. 4 zeigt den Body (Tulpe) 4, der an der Schraube (Pedikelschraube) 1 angelenkt werden kann/ist. Der hülsenförmige Body 4 hat dabei in Seitenansicht eine U-Form, d.h. eine im Wesentlichen hülsenförmige Gestalt mit zwei sich diametral gegenüberliegenden Längsschlitzen am proximalen Hülsenabschnitt und einer radialen Einschnürung am distalen Hülsenende. An der dem Werkzeug 2 zugewandten Seite des Body 4 (im Bereich des längsgeschlitzten Hülsenabschnitts) ist ein Innengewinde 30 ausgebildet, das komplementär zu dem Außengewinde 28 des werkzeugseitigen Gewindeteils 8 ist, also dieses aufnehmen kann.

An der distalen Seite des Bodys 4, also der Schraube 1 zugewandte Seite, ist eine Innenkalotte 32 ausgebildet, also eine bauchige Durchgangsbohrung. Wenn der Body 4 über die distale Spitze der Schraube 1 gestülpt und nach proximal in Richtung hin zum Schraubenkopf 36 geschoben wird, umfasst/umgreift die Innenkalotte 32 des Bodys 4 die Schraube 1 an ihrem oberen Ende (kugelförmiger Schraubenkopf 36). Somit ist der Body 4 beweglich an dem oberen Ende (Schraubenkopf 36) der Schraube 1 gehalten. Im verspannten Zustand, also wenn das Außengewinde 28 des Gewindeteils 8 in das Innengewinde 30 des Bodys 4 komplett eingeschraubt ist, drückt das Gewindeteil 8 das Werkzeug 2 bzw. den Werkzeugschaft 12 durch eine axiale Kraft auf den Gegenhalter 10, in die Schraube 1 (deren Schraubenkopf-seitiges Sackloch). Das Widerlager stellt dabei der Body 4 dar, welcher in Anlage mit der Schraube 1 bzw. deren Schraubenkopf ist.

Fig. 5 zeigt das Insert 6, das optional in dem Body 4 angeordnet werden kann. Das Insert 6 weist einen axialen Vorsprung mit einer Innenbohrung 34 auf, die passgenau mit dem Schaftabschnitt 14 unterhalb des Gegenhalters 10 des Werkzeugs 2 übereinstimmt, aber auch größer sein kann, und das in die Innenkalotte 32 des Body 4 eingesetzt werden kann oder ist, um sich gegen den Schraubenkopf 36 zu drücken. Der Gegenhalter 10 des Werkzeugs 2 stößt auch bei komplett eingedrehtem Gewindeteil 8 in dem Body 4 nicht an das Insert 6 an. Das Insert 6 bleibt somit auch im verspannten Zustand des Werkzeugs 2 mit dem Body 4 beweglich im Body 4. Das Insert 6 verspannt somit auch nicht im verspannten Zustand des Werkzeugs 2 mit der Schraube 1, bei eingedrehtem Gewindeteil 8 in den Body 4, und ist somit beweglich.

Fig. 6 zeigt den ball-/kugelförmigen Schraubenkopf 36 der zu implantierenden Schraube 1. In dem kugelartigen Schraubenkopf 36 ist ein Sackloch ausgeformt, das sich axial zur Schraubenachse A erstreckt und sich an der proximalen Seite des Schraubenkopfs 36 öffnet. Das Sackloch weist in einem distalen Drehmomenteinleitabschnitt 42 mindestens eine radial nach außen ragende Einbuchtung/Nut 44 und mindestens einen radial nach innen ragenden Zacken/Rippe/Steg 46 auf. Am Rand des Sacklochs ist ein (trichter-/kelchförmiger) Einführschrägenbereich (Anfasung) 41 ausgebildet, in dem schalen-/kalottenförmigen Einbuchtungen/Nuten 40 ausgeformt sind, die sich in Umfangsrichtung vorzugsweise mit gleichem Abstand aneinander reihen. Bevorzugt stellt somit jede Einbuchtung/Nut 40 im Einführschrägenbereich 41 einen eigenen/separaten Einführschrägenbereichausschnitt zusätzlich zu der mindestens einen Nut 44 im Drehmomenteinleitabschnitt 42 des Sacklochs dar. Bei einer Vielzahl von Einführschrägenbereichabschnitten (Einbuchtungen) 40 grenzt dabei ein Einführschrägenbereichsabschnitt 40 an den jeweils in Umfangsrichtung Benachbarten an, bzw. die Einführschrägenbereichsabschnitte 40 sind miteinander verschnitten und bilden eine gemeinsame Verschneidungskante aus. Die Verschneidungskante läuft in das Innere eines Werkzeugsaufnahmebereichs 42 des Sacklochs.

Die Einbuchtungen/Nuten 40 im Einführschrägenbereich 41 bilden dabei Form- und Kraftschlussbereiche für die vorstehend genannten semi-kegelförmigen axialen (und radialen) Vorsprünge am Wellen-/Schaftabsatz 16 des erfindungsgemäßen Werkzeugs 2. Erfindungsgemäß bilden die Einführschrägenbereichsabschnitte 40 Formschlussbereiche für die Zentrierfläche 16 des Werkzeugs 2.

Der Schraubenkopf 36 stellt das Widerlager, in anderen Worten die Anlagefläche, des Body 4 in dem verspannten Zustand dar, wenn das Gewindeteil 8 in den Body 4 eingeschraubt ist. Die Ausrichtung erfolgt über einen Kraft- und Formschluss von Zentrierfläche 16 bzw. den dort ausgebildeten Vorsprüngen 17 mit dem Einführschrägenbereich 41 bzw. den dort ausgebildeten Einbuchtungen 40.

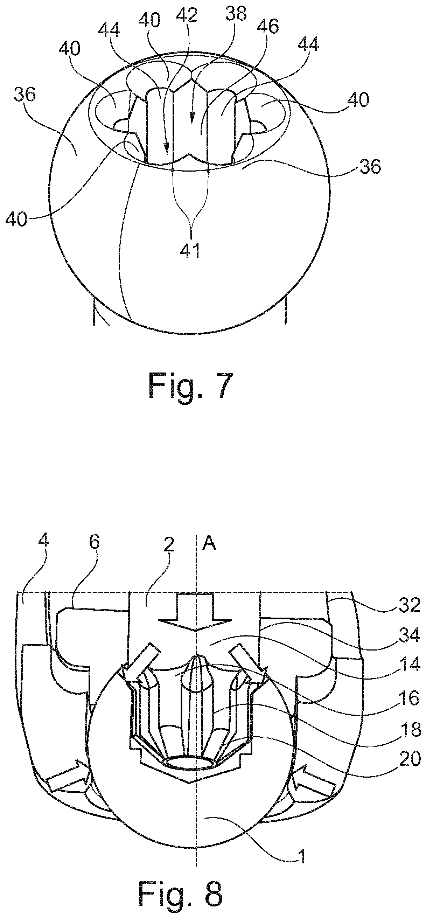

Fig. 7 zeigt den ball-/kugelförmigen Schraubenkopf 36 im Detail. Der Werkzeugaufnahmebereich/Drehmomenteinleitabschnitt 42 des Schraubenkopfs 36 bzw. des Sacklochs 38 weist axial zur Schraube 1 sich erstreckende, sowie radial nach außen eingebrachte Einbuchtungen/Nuten 44 auf. Die radial nach außen weisenden Einbuchtungen/Nuten 44 werden in Umfangsrichtung durch radial nach innen ragende Zacken/Stege/Rippen 46 getrennt. Am distalen Rand des Sacklochs 38 an dem Schraubenkopf 36 ist der Einführschrägenbereich 41 ausgebildet. Während bei herkömmlichen Schrauben der Einführschrägenbereich gleichmäßig/eben das gesamte Loch umgibt (nach Art einer einzigen gedrehten Anfasung) und in das Loch trichterartig hineinragt, ist in der Schraube 1 der vorliegenden Erfindung der Einführungsschrägenbereich 41 für jede Einbuchtung/Nut 44 quasi einzeln, d.h. durch in Umfangsrichtung beabstandeter sowie sich überlappender, jeweils trichterförmiger Einführschrägenbereichsabschnitte/Einbuchtungen 40 ausgestaltet. Dadurch ergibt sich in Draufsicht in etwa die Form eines mehrblättrigen Kleeblatts.

Fig. 8 zeigt das Werkzeug 2 in verspanntem Zustand gemäß einer vorteilhaften Ausführungsform. Das Werkzeug 2, befindet sich im Eingriff mit der Schraube 1 und der Schaftabschnitt 14 unterhalb/distal zum Gegenhalter 10 ist dabei in der Innenbohrung 34 in dem Insert 6 angeordnet, das wiederum in dem Body 4 angeordnet ist. Aus der Fig. 8 ist ersichtlich, dass das Insert 6 noch Spiel zu dem Body 4 hat und somit beweglich in diesem angeordnet ist, in der Längsachse des Werkzeugs 2 gesehen.

Das Sackloch 38 in der Schraube 1 bzw. im Schraubenkopf 36 ist tiefer als die Länge des Werkzeugenabschnitts/des Drehmomentübertragungsabschnitts 18 und dem daran sich anschließenden Endabschnitt 22 ausgehend von der distalen (abgeflachten Werkzeugspitze bis zum Wellen-/Schaftabsatz 16. In anderen Worten ausgedrückt steht das Werkzeug 2 in verspannten Zustand an der abgeflachten Werkzeugschaftspitze 22 nicht in Wirkeingriff/stirnseitiger Anlage mit dem Sackloch 38 der Schraube 1, während der Drehmomentübertragungsbereich 18 des Werkzeugs 2 in Wirkeingriff mit dem Drehmoment-Einleitabschnitt 42 des Sacklochs 38 steht. Das bedeutet, die Kraft, die von dem Werkzeug 2 auf Schraube 1 aufgebracht wird, wird teilweise (in Ergänzung zum Drehmomentübertragungsabschnitt 18) auch von den Vorsprüngen/Zentrierflächen 17 im Wellen-/Schaftabsatz 16 auf den Einführschrägenbereich 41 bzw. die dort ausgebildeten kalottenförmigen Einführschrägenbereichsabschnitte/Einbuchtungen 40 aufgebracht.

Die Einbuchtungen bzw. Axial-Nuten 44 im Drehmoment-Einleitabschnitt 42 des Sacklochs 38 bilden jeweils Form- und Kraftschlussbereiche für die Rippen/Stege 20 im Drehmoment-Übertragungsabschnitt 18 des Werkzeugs 2 und die (schalen/kalottenförmigen) Einführschrägenbereichsabschnitte/Einbuchtungen 40 bilden Form- und Kraftschlussbereiche für die Zentrierflächen/kugelförmigen Vorsprünge 17 des Werkzeugs 2. Die Zentrierflächen 17 des Werkzeugs 2 sind analog zu den Einführschrägenbereichsabschnitten 40 des Werkzeugs 2 ausgebildet.

Die Erfindung betrifft zusammenfassend ein Implantationsset zum Implantieren einer Schraube 1 in einen menschlichen oder tierischen Körper, aufweisend ein Werkzeug 2 und eine zum Aufnehmen des Werkzeugs 2 ausgelegte Schraube 1, wobei die Schraube (1) einen Werkzeugaufnahmebereich (42) aufweist, in den oder auf den ein Drehmomentübertragungsbereich (18) des Werkzeugs (2) zur Drehmomentübertragung einsetzbar/aufsetzbar oder eingesetzt/aufgesetzt ist, wobei sich an dem Werkzeugaufnahmebereich (42) zumindest ein Einführschrägenbereich (41) zum Führen des Werkzeugs (2) während des Einsetzens der Schraube (1) ausbildet, dadurch gekennzeichnet, dass das Werkzeug (2) zumindest eine Zentrierfläche (16) ausbildet, die mit dem Einführschrägenbereich (41) so abgestimmt ist, dass beim in Kontakt gelangen der Zentrierfläche (16) mit dem Einführschrägenbereich (41) ein Form- und Kraftschluss dazwischen entsteht.

### Bezugszeichenliste

- 1: Schraube
- 2: Werkzeug
- 4: Body
- 6: Insert
- 8: Gewindeteil
- 10: Gegenhalter
- 12: Schraubendreherschaft
- 14: Schaftabschnitt unterhalb des Gegenhalters
- 16: Wellen-/Schaftabsatz
- 17: Einbuchtungen/Zentrierflächen
- 18: Drehmomentübertragungsbereich/-abschnitt
- 20: Rippen
- 22: Werkzeugschaft-Endabschnitt
- 24: Gewindeteilloch
- 26: Schaft oberhalb des Gewindes
- 28: Außengewinde des Gewindeteils
- 30: Innengewinde Body
- 32: Innenkalotte
- 34: Innenbohrung
- 36: Ballartiger Schraubenkopf
- 38: Loch
- 40: kalottenförmige Einbuchtungen
- 41: Einführschrägenbereich
- 42: Drehmomenteinleitabschnitt einer Werkzeugaufnahme
- 44: Einbuchtungen/Axialnuten
- 46: Zacken/Rippen/Stege

## Patentansprüche

1. Implantationsset zum Implantieren einer Schraube (1) in einen menschlichen oder tierischen Körper, aufweisend
- ein Werkzeug (2) mit einem Werkzeugschaft (12) und
- eine für ein Aufnehmen des Werkzeugs (2) ausgelegte Schraube (1), wofür
- die Schraube (1) eine Werkzeugaufnahme vorzugsweise in Form eines Sacklochs (38) mit einem Drehmomenteinleitabschnitt (42) aufweist, in den oder auf den ein Drehmomentübertragungsabschnitt (18) am distalen Endbereich des Werkzeugschafts (12) zur Drehmomentübertragung einsetzbar/aufsetzbar oder eingesetzt/aufgesetzt ist, wobei die Werkzeugaufnahme der Schraube (1) proximal zum Drehmomenteinleitabschnitt (42) einen Einführschrägenbereich (41) zum Führen und/oder Zentrieren des Werkzeugschafts (12) während des Ein-/Aufsetzens in/auf die Schraube (1) ausbildet, wobei
- der Werkzeugschaft (12) eine Zentrierfläche (16) proximal zum Drehmomentübertragungsabschnitt (18) ausbildet, die auf den Einführschrägenbereich (41) so abgestimmt ist, dass beim in Kontakt gelangen der Zentrierfläche (16) mit dem Einführschrägenbereich (41) dazwischen ein Form- und vorzugsweise ein Kraftschluss in Umfangsrichtung zusätzlich oder alternativ zu dem Formschluss zwischen dem Drehmomentübertragungsabschnitt (18) und dem Drehmomenteinleitabschnitt (42) entsteht, wobei der Werkzeugschaft (12) einen radial vorragenden Wellen-/Schaftring oder Absatz ausbildet oder hat, der den Drehmomentübertragungsabschnitt (18) in axialer Richtung proximal begrenzt und an dessen distaler Seite die Zentrierfläche (16) ausgebildet ist, **dadurch gekennzeichnet, dass** in dem Wellen-/Schaftring eine Anzahl gleichmäßig umfangsbeabstandeter, axial und auch radial vorragender, kugelförmiger Vorsprünge (17) ausgeformt ist, und der Einführschrägenbereich (41), der am proximalen Ende des Drehmomenteinleitabschnitts (42) angeordnet ist, eine Anzahl gleichmäßig umfangsbeabstandeter, axial und auch radial sich ausdehnender, kugelkalottenförmiger Einbuchtungen oder Nuten (40) ausbildet, die mit den kugelförmigen Vorsprüngen (17) an der Zentrierfläche (16) in Formschuss-Eingriff bringbar sind, wobei der Übergang zwischen der Zentrierfläche (16) und dem Drehmomentübertragungsabschnitt (18) durch die kugelförmigen Vorsprünge (17) fließend ausgebildet ist.

2. Implantationsset nach Anspruch 1, **dadurch gekennzeichnet, dass** der Drehmomentübertragungsabschnitt (18) am Werkzeugschaft (12) des Werkzeugs (2) und der Drehmomenteinleitabschnitt (42) an/in der Werkzeugaufnahme der Schraube (1) jeweils zumindest eine oder mehrere in Umfangsrichtung vorzugsweise gleichmäßig beabstandete sowie axial sich erstreckende Rippen oder Stege (20, 46) und zumindest eine oder mehrere in Umfangsrichtung vorzugsweise gleichmäßig beabstandete sowie axial sich erstreckende Nuten (44) haben oder vorzugsweise jeweils eine in Wirkeingriff miteinander bringbare Torx-/Vielnut-/Inbusform ausbilden.

3. Implantationsset nach einem der vorstehenden Ansprüche, **dadurch gekennzeichnet, dass** sich die an einer zylindrischen Außenfläche eines distalen Schaftabschnitts (14) des Werkzeugs (2), welcher den Drehmomentübertragungsabschnitt (18) bildet, axial sich erstreckenden, radial nach außen vorstehenden Rippen oder Stege (20) über eine distale kegelstumpfförmige Außenfläche einer Spitze (22) des Werkzeugs (2) hinweg weiter in Richtung distal erstrecken.

4. Implantationsset nach einem der vorstehenden Ansprüche, **dadurch gekennzeichnet, dass** auf dem Werkzeugschaft (12) ein hülsenförmiges Gewindeteil (8) relativ beweglich aufgesetzt ist, das ein Außengewinde (28) an seinem distalen Ende aufweist und an der Schraube (1) ein Body oder Tulpe (4) beweglich gelagert ist, die einen Schraubenkopf (38) umgreift und an ihrem proximalen längsgeschlitzten Endabschnitt ein Innengewinde (30) ausbildet, das mit dem Außengewinde (28) des hülsenförmigen Gewindeteils (8) in Eingriff bringbar ist.

5. Implantationsset nach Anspruch 4, **dadurch gekennzeichnet, dass** an dem Werkzeugschaft (12) ein Ring- oder Wellenabsatz-förmiger Gegenhalter (10) ausgebildet oder fixiert ist, der dafür konfiguriert und/oder angepasst ist, in Längsschlitze in dem Body/Tulpe (4) axial eingeführt zu werden, um auf den Body/Tulpe (4) ein Drehmoment, insbesondere ein Festhaltemoment aufzubringen, wobei das hülsenförmige Gewindeteil (8) proximal zum Gegenhalter (10) angeordnet ist, derart, dass sich dessen Außengewinde (28) auf Seiten des Gegenhalters (10) befindet und das hülsenförmige Gewindeteil (8) axial mit dem Gegenhalter (10) in Richtung distal in Anlage bringbar ist, wenn das hülsenförmige Gewindeteil (8) in den Body/Tulpe (4) unter dessen Festhalten mittels des Gegenhalters (10) eingedreht wird.

6. Medizinisches Werkzeug (2) zum Einschrauben einer Schraube (1) vorzugsweise Pedikelschraube mit einem Body oder Tulpe (4) weiter vorzugsweise der polyaxialen Bauart in einen menschlichen oder tierischen Körper mit einem am distalen Endbereich eines Werkzeugschafts (12, 14) ausgebildeten Drehmomentübertragungsabschnitt (18), der dafür konfiguriert und/oder angepasst ist, in oder auf einen Drehmomenteinleitabschnitt (42) einer Werkzeugaufnahme der Schraube (1) eingesteckt/aufgesteckt zu werden, wobei am Werkzeugschaft (12, 14) ein radial vorragender Wellen-/Schaftring oder Absatz ausgebildet oder angeordnet ist, der proximal zum Drehmomentübertragungsabschnitt (18) positioniert ist und den Drehmomentübertragungsabschnitt (18) proximal begrenzt, wobei an der distalen Seite des Wellen-/Schaftabsatzes eine Zentrierfläche (16) ausgebildet ist, **dadurch gekennzeichnet, dass** die Zentrierfläche eine Anzahl gleichmäßig umfangsbeabstandeter, axial und auch radial vorragender kugelförmiger Vorsprünge (17) aufweist oder bildet, wobei der Übergang zwischen der Zentrierfläche (16) und dem Drehmomentübertragungsabschnitt (18) durch die Vorsprünge (17) fließend ausgebildet ist..

7. Werkzeug (2) nach Anspruch 6, **dadurch gekennzeichnet, dass** der Drehmomentübertragungsabschnitt (18) eine Anzahl von gleichmäßig umfangsbeabstandeter sowie axial sich erstreckender und radial vorragender Rippen/Stege (20) hat, die in axialer Verlängerung zu den in der Zentrierfläche (16) ausgebildeten Vorsprüngen (17) angeordnet sind, derart, dass die in der Zentrierfläche (16) ausgebildeten Vorsprünge (17) die jeweils zugeordneten Rippen/Stege (20) radial überragen.

## Claims

1. An implantation set for implanting a screw (1) in a human or animal body, comprising
- a tool (2) having a tool shank (12) and
- a screw (1) designed to receive the tool (2), for which
- the screw (1) comprises a tool mount preferably in the form of a blind hole (38) comprising a torque introduction portion (42) into which or onto which a torque transmission portion (18) on a distal end region of the tool shank (12) can be inserted/placed or is inserted/placed to transmit torque, wherein the tool mount of the screw (1) forms, proximal to the torque introduction portion (42), an inclined insertion region (41) for guiding and/or centering the tool shank (12) during inserting it in/placing it on the screw (1),wherein
- the tool shank (12) forms a centering surface (16) proximal to the torque transmission portion (18), which is matched to the inclined insertion region (41) in such a way that when the centering surface (16) comes into contact with the inclined insertion region (41) therebetween, a form fit and preferably a force fit connection is created in a circumferential direction in addition to or as an alternative to the form fit connection between the torque transmission portion (18) and the torque introduction portion (42), wherein
the tool shank (12) forms or comprises a radially protruding shaft/shank ring or shoulder which delimits the torque transmission portion (18) proximally in an axial direction and on the distal side of which the centering surface (16) is formed, **characterized in that** a number of equally circumferentially spaced, axially and also radially projecting, spherical protrusions (17) are formed in the shaft/shank ring, and the inclined insertion region (41), which is arranged at the proximal end of the torque introduction portion (42), forms a number of equally circumferentially spaced, axially and also radially extending, spherical calotte-shaped indentations or grooves (40) which can be brought into form-fitting engagement with the spherical protrusions (17) on the centering surface (16), wherein the transition between the centering surface (16) and the torque transmission portion (18) is formed smoothly by the spherical protrusions (17).

2. The implantation set according to claim 1, **characterized in that** the torque transmission portion (18) on the tool shank (12) of the tool (2) and the torque introduction portion (42) on/in the tool shank of the screw (1) each have at least one or more circumferentially preferably equally spaced and axially extending ribs or webs (20, 46) and at least one or more circumferentially preferably equally spaced and axially extending grooves (44), or preferably each form a Torx/multi groove/hex shape which can be brought into operative engagement with each other.

3. The implantation set according to any one of the preceding claims, **characterized in that** the ribs or webs (20) which extend axially and protrude radially outward on a cylindrical outer surface of a distal shank portion (14) of the tool (2), forming the torque transmission portion (18), extend beyond a distal frustoconical outer surface of a tip (22) of the tool (2) in distal direction.

4. The implantation set according to one of the preceding claims, **characterized in that** a sleeve-shaped threaded part (8) is placed on the tool shank (12) so as to be movable relative thereto, said threaded part having an external thread (28) at its distal end and a body or tulip (4) being movably supported on the screw (1), which encompasses a screw head (38) and forms an internal thread (30) at its proximal end portion provided with longitudinal slits, which can be brought into engagement with the external thread (28) of the sleeve-shaped threaded part (8).

5. The implantation set according to claim 4, **characterized in that** a ring-shaped or shaft shoulder-shaped counterholder (10) is formed or fixed on the tool shank (12), and configured and/or adapted to be axially inserted into longitudinal slits in the body/tulip (4) in order to apply a torque, in particular a retaining torque, onto the body/tulip (4), wherein the sleeve-shaped threaded part (8) is arranged proximally to the counterholder (10) such that its external thread (28) is located on the side of the counterholder (10) and the sleeve-shaped threaded part (8) is axially engageable with the counterholder (10) in the distal direction when the sleeve-shaped threaded part (8) is screwed into the body/tulip (4) while retaining it by means of the counterholder (10).

6. A medical tool (2) for screwing a screw (1) preferably pedicle screw with a body or tulip (4) further preferably of the polyaxial type in a human or animal body, comprising a torque transmission portion (18) which is formed on the distal end region of a tool shank (12, 14) and configured and/or adapted to be inserted in/placed on a torque introduction portion (42) of a tool mount of the screw (1), wherein a radially protruding shaft/shank ring or shoulder is formed or arranged on the tool shank (12, 14) which is positioned proximally to the torque transmission portion (18) and delimits the torque transmission portion (18) proximally, wherein a centering surface (16) is formed on the distal side of the shaft/shank shoulder, **characterized in that** the centering surface has or forms a number of equally circumferentially spaced, axially and also radially projecting spherical protrusions (17), wherein the transition between the centering surface (16) and the torque-transmitting section (18) is formed smoothly by the protrusions (17).

7. The tool (2) according to claim 6, **characterized in that** the torque transmission portion (18) has a number of equally circumferentially spaced as well as axially extending and radially protruding ribs/webs (20) arranged in axial extension to the protrusions (17) formed in the centering surface (16), such that the protrusions (17) formed in the centering surface (16) extend radially beyond the respectively associated ribs/webs (20).

## Revendications

1. Ensemble d'implantation permettant l'implantation d'une vis (1) dans un corps humain ou animal, présentant
- un outil (2) comportant une tige d'outil (12) et
- une vis (1) conçue pour une réception de l'outil (2), moyennant quoi
- la vis (1) présente un réceptacle d'outil, de préférence sous la forme d'un trou borgne (38) comportant une section d'introduction de couple (42) dans laquelle ou sur laquelle une section de transmission de couple (18) peut être installée ou est installée au niveau de la zone d'extrémité distale de la tige d'outil (12) pour la transmission de couple, le réceptacle d'outil de la vis (1) formant, de manière proximale par rapport à la section d'introduction de couple (42), une zone inclinée d'introduction (41) pour le guidage et/ou le centrage de la tige d'outil (12) pendant l'installation dans/sur la vis (1),
- la tige d'outil (12) formant une surface de centrage (16) proximale par rapport à la section de transmission de couple (18) et adaptée à la zone inclinée d'introduction (41) de sorte que, lorsque la surface de centrage (16) entre en contact avec la zone inclinée d'introduction (41), une liaison par complémentarité de forme et de préférence une liaison à force complémentaire à la liaison par complémentarité de forme ou pouvant remplacer celle-ci est établie dans la direction circonférentielle entre la section de transmission de couple (18) et la section d'introduction de couple (42), la tige d'outil (12) formant ou possédant un anneau ou un épaulement d'arbre/de tige faisant saillie radialement, lequel délimite de manière proximale la section de transmission de couple (18) dans la direction axiale et la surface de centrage (16) étant réalisée sur le côté distal de celui-ci, **caractérisé en ce qu'**un certain nombre de saillies (17) sphériques régulièrement espacées circonférentiellement et faisant saillie axialement et également radialement sont moulées dans l'anneau d'arbre/de tige, et la zone inclinée d'insertion (41), laquelle est disposée au niveau de l'extrémité proximale de la section d'introduction de couple (42), forme un certain nombre d'indentations ou de rainures (40) en forme de calotte sphérique, régulièrement espacées circonférentiellement et étendues axialement et également radialement, lesquelles peuvent être amenées en prise par complémentarité de forme avec les saillies (17) sphériques sur la surface de centrage (16), la transition entre la surface de centrage (16) et la section de transmission de couple (18) étant réalisée de manière à être fluide à l'aide des saillies (17) sphériques.

2. Ensemble d'implantation selon la revendication 1, **caractérisé en ce que** la section de transmission de couple (18) sur la tige d'outil (12) de l'outil (2) et la section d'introduction de couple (42) sur/dans le réceptacle d'outil de la vis (1) possèdent respectivement au moins une ou plusieurs nervures ou barrettes (20, 46), de préférence régulièrement espacées dans la direction circonférentielle et s'étendant axialement, et au moins une ou plusieurs rainures (44), de préférence régulièrement espacées dans la direction circonférentielle et s'étendant axialement, ou forment de préférence respectivement une forme de type Torx/à plusieurs rainures/de type Inbus pouvant être amenées en prise fonctionnelle l'une avec l'autre.

3. Ensemble d'implantation selon l'une des revendications précédentes, **caractérisé en ce que** les nervures ou barrettes (20) faisant saillie radialement vers l'extérieur et s'étendant axialement sur une surface externe cylindrique d'une section de tige distale (14) de l'outil (2), laquelle section de tige distale forme la section de transmission de couple (18), s'étendent plus loin dans la direction distale sur une surface externe tronconique distale d'une pointe (22) de l'outil (2).

4. Ensemble d'implantation selon l'une des revendications précédentes, **caractérisé en ce qu'**une partie filetée (8) en forme de douille présentant un filetage externe (28) au niveau de son extrémité distale est installée de manière relativement mobile sur la tige d'outil (12) et un corps ou une tulipe (4) est monté de manière mobile sur la vis (1), entoure une tête de vis (38) et forme un filetage interne (30) au niveau de sa section d'extrémité proximale fendue longitudinalement, lequel filetage interne peut être amené en prise avec le filetage externe (28) de la partie filetée (8) en forme de douille.

5. Ensemble d'implantation selon la revendication 4, **caractérisé en ce qu'**un contre-support (10) en forme d'anneau ou d'épaulement d'arbre est réalisé ou fixé sur la tige d'outil (12), lequel contre-support est conçu et/ou adapté pour être introduit axialement dans des fentes longitudinales dans le corps/la tulipe (4) afin d'appliquer un couple, en particulier un couple de maintien, sur le corps/la tulipe (4), la partie filetée (8) en forme de douille étant disposée de manière proximale par rapport au contre-support (10) de telle sorte que son filetage externe (28) se trouve sur des côtés du contre-support (10) et la partie filetée (8) en forme de douille pouvant être amenée axialement en appui contre le contre-support (10) dans la direction distale lorsque la partie filetée (8) en forme de douille est vissée dans le corps/la tulipe (4) lors de son maintien au moyen du contre-support (10).

6. Outil médical (2) permettant de visser une vis (1), de préférence une vis pédiculaire comportant un corps ou une tulipe (4), plus préférablement de type polyaxiale, dans un corps humain ou animal, comportant une section de transmission de couple (18) réalisée au niveau de la zone d'extrémité distale d'une tige d'outil (12, 14) et conçue et/ou adaptée pour être installée dans ou sur une section d'introduction de couple (42) d'un réceptacle d'outil de la vis (1),
un anneau ou un épaulement d'arbre/de tige faisant saillie radialement étant réalisé ou disposé sur la tige d'outil (12, 14), lequel étant positionné de manière proximale par rapport à la section de transmission de couple (18) et délimitant la section de transmission de couple (18) de manière proximale, une surface de centrage (16) étant réalisée sur le côté distal de l'épaulement d'arbre/de tige, **caractérisé en ce que** la surface de centrage présente ou forme un certain nombre de saillies (17) sphériques régulièrement espacées circonférentiellement et faisant saillie axialement et également radialement, la transition entre la surface de centrage (16) et la section de transmission de couple (18) étant réalisée de manière à être fluide à l'aide des saillies (17).

7. Outil (2) selon la revendication 6, **caractérisé en ce que** la section de transmission de couple (18) possède un certain nombre de nervures/barrettes (20) régulièrement espacées circonférentiellement, s'étendant axialement et faisant saillie radialement, lesquelles sont disposées dans le prolongement axial par rapport aux saillies (17) réalisées dans la surface de centrage (16) de telle sorte que les saillies (17) réalisées dans la surface de centrage (16) font saillie radialement au-delà des nervures/barrettes (20) respectivement associées.
